# EUROPEAN PATENT APPLICATION

(11) **EP 4 424 329 A1**
(43) Date of publication of application: **04.09.2024**
(21) Application number: 22887455.8
(22) Date of filing: 18.10.2022
(51) Int. Cl.: A61K 48/00, A61K 47/02, C09K 11/65, A61K 9/51, A61P 31/14

(54) **GENE DELIVERY COMPOSITION COMPRISING NITROGEN-DOPED GRAPHENE QUANTUM DOTS**

(30) Priority: 25.10.2021 KR 20210142996; 31.12.2021 KR 20210194014
(71) Applicant: Graphene Square Chemical Inc., Pohang-si, Gyeongsangbuk-do 37673 (KR); Seoul National University R&DB Foundation, Seoul 08826 (KR)
(72) Inventor: HONG, Byung Hee, Suwon-Si, Gyeonggi-do 16420 (KR); AHN, Minchul, Seoul 08075 (KR); PARK, Jong Bo, Seoul 08539 (KR)
(74) Representative: Nederlandsch Octrooibureau
(86) International application number: PCT/KR2022/015792
(87) International publication number: WO 2023/075259

(57) **Abstract**

The present invention relates to a gene delivery composition comprising nitrogen-doped graphene quantum dots as an active ingredient, the nitrogen-doped graphene quantum dots of the present invention exhibiting excellent safety and low cytotoxicity in room temperature, and having positively charged surfaces, thereby binding with negatively charged genes through electrostatic attractive force and thus being capable of delivering the genes into cells, and exhibiting excellent gene delivery efficiency and transfection efficiency, thereby being expected to be usefully employable for a gene delivery platform for gene therapy.

## Description

### [Technical Field]

The present invention relates to a gene delivery composition comprising nitrogen-doped graphene quantum dots.

This application claims priority to and the benefit of Korean Patent Application Nos. 10-2021-0142996 and 10-2021-0194014 filed on October 25, 2021 and December 31, 2021, respectively, and all the contents disclosed in the specification and drawings of the applications are incorporated in this application.

### [Background Art]

Gene therapy is used to treat diseases by delivering therapeutic genes to desired organs in the body and causing new proteins to be expressed within cells. In recent years, gene therapy has been widely reported to have effective treatment effects for various diseases such as viral diseases, cancer, and the like. As the first gene therapy product was developed and commercialized in Europe, research on gene therapy products is actively underway in Korea. In fact, as several gene therapy products have exhibited excellent therapeutic effects in clinical studies, expectations for the development of gene therapy products are high not only in Korea but also around the world. However, despite receiving this attention, it is extremely rare for gene therapy products to be actually commercialized because they do not show outstanding efficacy in early clinical trials. The main reasons for this include the safety of gene therapy products and their low gene delivery efficiency to target cells. Therefore, for effective gene therapy, it is essential to develop a gene delivery platform that can safely deliver therapeutic genes to desired target cells in order to achieve high expression efficiency.

Currently, viral gene delivery platforms having high delivery efficiency are being used, but viral vectors such as a retrovirus, an adenovirus, and an adeno-associated virus have a complicated construction process. In addition, there are many limitations to their application to the human body due to safety issues such as immunogenicity, the possibility of infection, inflammation, non-specific DNA insertion, and the like and the limited size of nucleic acids that can be accommodated. Accordingly, non-viral gene delivery platforms are currently attracting attention as an alternative to the viral gene delivery platforms. The non-viral gene delivery platforms have the advantages of enabling repeated administration with minimal immune response and specific delivery to specific cells and having excellent safety and storage stability and easy mass production. Examples of these non-viral gene delivery platforms include a cationic liposome series, for example, N-[1-(2,3-dioleyloxy)propyl]-N,N,N-triethylammonium chloride (DOTMA), alkylammonium, cationic cholesterol derivatives, gramicidin, and the like. However, such non-viral gene delivery platforms also have disadvantages in that they exhibit significantly high cytotoxicity due to poor biocompatibility and non-biodegradability and have low blood stability and poor gene delivery efficiency.

Therefore, there is an urgent need for the development of a gene delivery platform that exhibits excellent delivery efficiency while overcoming the above shortcomings.

### [Disclosure]

### [Technical Problem]

The present inventors have made extensive efforts to develop an optimized gene delivery platform that is safe and exhibits excellent delivery efficiency and found that nitrogen-doped graphene quantum dots synthesized from citric acid and polyethylenimine as precursors have excellent stability and low cytotoxicity at room temperature while exhibiting similar or better gene delivery efficiency and transfection efficiency than existing gene delivery platforms. Therefore, the present invention has been completed based on the above facts.

Accordingly, it is an object of the present invention to provide a gene delivery composition comprising nitrogen-doped graphene quantum dots (NGQDs) or a salt thereof as an active ingredient.

It is another object of the present invention to provide a method of preparing nitrogen-doped graphene quantum dots.

It is still another object of the present invention to provide nitrogen-doped graphene quantum dots prepared by the preparation method according to the present invention.

However, the technical objects to be achieved by the present invention are not limited to the above-described technical objects, and other objects which are not mentioned above will be clearly understood from the following detailed description by those skilled in the art to which the present invention pertains.

### [Technical Solution]

To solve the above problems, according to an aspect of the present invention, there is provided a gene delivery composition copmrising nitrogen-doped graphene quantum dots (NGQDs) or a pharmaceutically acceptable salt thereof as an active ingredient.

According to one exemplary embodiment of the present invention, the nitrogen-doped graphene quantum dots may have a zeta potential ranging from 0.1 to 20 mV, but the present invention is not limited thereto.

According to another exemplary embodiment of the present invention, the nitrogen-doped graphene quantum dots may have a diameter of 1 to 20 nm, but the present invention is not limited thereto.

According to still another exemplary embodiment of the present invention, the gene may be at least one selected from the group consisting of gDNA, cDNA, pDNA, mRNA, tRNA, rRNA, siRNA, shRNA, miRNA, a DNA-RNA hybrid, and a ribozyme, but the present invention is not limited thereto.

According to yet another exemplary embodiment of the present invention, the nitrogen-doped graphene quantum dots and the gene may be bound at a ratio of 1 to 50:1, but the present invention is not limited thereto.

According to yet another exemplary embodiment of the present invention, the nitrogen-doped graphene quantum dots and the gene may be bound through electrostatic interaction, but the present invention is not limited thereto.

According to another aspect of the present invention, there is provided a method of preparing nitrogen-doped graphene quantum dots, which includes: subjecting a mixed solution of citric acid and polyethylenimine (PEI) to a hydrothermal reaction.

According to one exemplary embodiment of the present invention, the hydrothermal reaction may be carried out by microwave irradiation, but the present invention is not limited thereto.

According to another exemplary embodiment of the present invention, the citric acid and the polyethylenimine may be mixed in a weight ratio of 1 to 10:1, but the present invention is not limited thereto.

According to still another aspect of the present invention, there are provided nitrogen-doped graphene quantum dots prepared by the preparation method according to the present invention.

According to yet another aspect of the present invention, there is provided a gene delivery method comprising: administering a composition, which includes, as an active ingredient, nitrogen-doped graphene quantum dots or a salt thereof bound to a gene to be delivered, to a subject in need thereof.

According to yet another aspect of the present invention, there is provided a use of the composition comprising the nitrogen-doped graphene quantum dots as an active ingredient for gene delivery.

According to yet another aspect of the present invention, there is provided a gene delivery method comprising: administering nitrogen-doped graphene quantum dots or a pharmaceutically acceptable salt thereof to which a target gene is bound to a subject in need thereof.

According to yet another aspect of the present invention, there is provided a use of the nitrogen-doped graphene quantum dots or a pharmaceutically acceptable salt thereof for gene delivery.

According to yet another aspect of the present invention, there is provided a use of the nitrogen-doped graphene quantum dots or a pharmaceutically acceptable salt thereof for the preparation of a gene carrier.

### [Advantageous Effects]

Nitrogen-doped graphene quantum dots of the present invention exhibit excellent safety and low cytotoxicity at room temperature, and have a surface with a positive charge, and thus can bind to negatively charged genes through electrostatic attraction to deliver the genes into cells. Also, the nitrogen-doped graphene quantum dots of the present invention can be usefully used for a gene delivery platform for gene therapy because the nitrogen-doped graphene quantum dots of the present invention exhibit excellent gene delivery efficiency and transfection efficiency.

### [Description of Drawings]

FIG. 1 shows the results of analyzing the characteristics of nitrogen-doped graphene quantum dots: FIG. 1A is a diagram showing a TEM image of the nitrogen-doped graphene quantum dots (scale bar = 20 nm), FIG. 1B is a diagram showing the size distribution of the nitrogen-doped graphene quantum dots, and FIG. 1C is a diagram showing the zeta potential of the nitrogen-doped graphene quantum dots.
FIG. 2 is a diagram showing the FT-IR spectrum of nitrogen-doped graphene quantum dots.
FIG. 3 is a diagram showing the FT-IR spectrum of a precursor for nitrogen-doped graphene quantum dots.
FIG. 4 is a diagram showing the XPS C1s spectrum (left) and the XPS N1s spectrum (right) of nitrogen-doped graphene quantum dots.
FIG. 5 is a diagram showing the Raman spectrum of nitrogen-doped graphene quantum dots.
FIG. 6 shows the results of determining the fluorescence and absorption characteristics of nitrogen-doped graphene quantum dots; FIG. 6A is a diagram showing the UV-Vis absorption spectrum of the nitrogen-doped graphene quantum dots, FIG. 6B is a diagram showing optical and fluorescence images of nitrogen-doped graphene quantum dots at 365 nm excitation, and FIG. 6C is a diagram showing the emission spectrum of nitrogen-doped graphene quantum dots at 365 nm excitation.
FIG. 7 shows the loading capacity of nitrogen-doped graphene quantum dots: FIG. 7A is a diagram showing the mRNA loading capacity and FIG. 7B is a diagram showing the pDNA loading capacity.
FIG. 8 is a diagram showing the results of confirming the cytotoxicity of nitrogen-doped graphene quantum dots.
FIG. 9 is a diagram showing the results of observing nitrogen-doped graphene quantum dots under a fluorescence microscope after HeLa cells are treated with a nitrogen-doped graphene quantum dot-mRNA complex in order to confirm the mRNA transfection efficiency of nitrogen-doped graphene quantum dots (scale bar = 200 µm).
FIGS. 10A and 10B are diagrams showing the results of flow cytometry to confirm the mRNA transfection efficiency of nitrogen-doped graphene quantum dots.
FIG. 11 is a diagram showing the results of observing nitrogen-doped graphene quantum dots under a fluorescence microscope after HeLa cells are treated with a nitrogen-doped graphene quantum dot-pDNA complex in order to confirm the pDNA transfection efficiency of nitrogen-doped graphene quantum dots (scale bar = 200 µm).
FIGS. 12A and 12B are diagrams showing the results of flow cytometry to confirm the pDNA transfection efficiency of nitrogen-doped graphene quantum dots.

### [Best Mode]

The present inventors confirmed that nitrogen-doped graphene quantum dots prepared using citric acid and polyethylenimine as precursors exhibited excellent transfection efficiency. Therefore, the present invention was completed based on this finding.

Specifically, according to one experimental example of the present invention, it was confirmed that the nitrogen-doped graphene quantum dots had an average diameter of 7.03 nm ± 0.27 nm, contained a graphene domain and functional groups such as carboxylic acid and amine, and had a surface with a positive charge (1.91 ± 1.77 mV) (see Experimental Example 1).

According to another experimental example of the present invention, it was confirmed that the loading capacity of the nitrogen-doped graphene quantum dots was NGQDs:gene = 20:1 (see Experimental Example 2).

According to still another experimental example of the present invention, it was confirmed that the nitrogen-doped graphene quantum dots exhibited low cytotoxicity (see Experimental Example 3).

According to yet another experimental example of the present invention, it was confirmed that the nitrogen-doped graphene quantum dots exhibited similar or better transfection efficiency for mRNA and pDNA than commercially available gene delivery platforms (see Experimental Example 5).

Hereinafter, the present invention will be described in detail.

The present invention provides a gene delivery composition comprising nitrogen-doped graphene quantum dots (NGQDs) or a pharmaceutically acceptable salt thereof as an active ingredient.

As used in the present invention, the term "graphene" refers to a polycyclic aromatic molecule formed by covalently linking a plurality of carbon atoms to each other. In this case, the carbon atoms linked via a covalent bond form a 6-membered ring as a basic repeating unit, but it is also possible to further include a 5-membered ring and/or a 7-membered ring.

As used in the present invention, the term "graphene quantum dots (GQDs)" refer to nano-sized graphene fragments. According to one embodiment of the present invention, the graphene quantum dots may be nitrogen-doped graphene quantum dots or a pharmaceutically acceptable salt thereof.

The "nitrogen-doped graphene quantum dots (NGQDs)" refer to graphene quantum dots including nitrogen in addition to typical graphene quantum dots. According to one embodiment of the present invention, the nitrogen-doped graphene quantum dots may be prepared using citric acid and polyethylenimine (PEI) as precursors, but the present invention is not limited thereto.

As used in the present invention, the term "nitrogen doping" refers to a process of adding nitrogen between carbon atoms constituting graphene.

In the present invention, the doped nitrogen of the nitrogen-doped graphene quantum dots may be in a pyridinic-N configuration, a pyrrolic-N configuration, and/or a graphitic-N configuration. In this case, the pyridinic-N configuration may account for 30 to 35%, but the present invention is not limited thereto.

Because the nitrogen-doped graphene quantum dots according to the present invention have a surface with a positive charge, the positive charge generated on the surface may be used to bind to a negatively charged gene in order to deliver the gene into cells.

In the present invention, the nitrogen-doped graphene quantum dots may have a zeta potential ranging from 0.1 to 20 mV, 0.5 to 20 mV, 1 to 20 mV, 3 to 20 mV, 5 to 20 mV, 8 to 20 mV, 10 to 20 mV, 10 to 18 mV, 10 to 15 mV, 10 to 13 mV, 0.1 to 10 mV, 0.3 to 10 mV, 0.5 to 10 mV, 0.8 to 10 mV, 1 to 10 mV, 3 to 10 mV, 5 to 10 mV, 5.5 to 10 mV, 6 to 10 mV, 6 to 9 mV, 6 to 7 mV, 0.1 to 6 mV, 0.13 to 6 mV, 0.14 to 6 mV, 0.15 to 6 mV, 0.5 to 6 mV, 0.8 to 6 mV, 1 to 6 mV, 2 to 6 mV, 3 to 6 mV, 3.5 to 6 mV, 4 to 6 mV, 4.5 to 6 mV, 5 to 6 mV, 5.5 to 6 mV, 0.1 to 5.5 mV, 0.13 to 5 mV, 0.14 to 4.5 mV, 0.14 to 4 mV, 0.14 to 3.8 mV, 0.14 to 3.7 mV, 0.15 to 3.7 mV, 0.18 to 3.7 mV, 0.2 to 3.7 mV, 0.25 to 3.7 mV, 0.3 to 3.7 mV, 0.5 to 3.7 mV, 0.8 to 3.7 mV, 1 to 3.7 mV, 1.5 to 3.7 mV, 2 to 3.7 mV, 2.5 to 3.7 mV, or 3 to 3.7 mV. According to one embodiment of the present invention, the nitrogen-doped graphene quantum dots may have a zeta potential ranging from 0.14 to 3.68 mV, but the present invention is not limited thereto.

In the present invention, the nitrogen-doped graphene quantum dots may have a diameter of 1 to 20 nm, 1 to 18 nm, 1 to 16 nm, 1 to 14 nm, 1 to 12 nm, 1 to 10 nm, 1 to 8 nm, 1 to 6 nm, 1 to 4 nm, 1 to 2 nm, 2 to 19 nm, 2 to 17 nm, 2 to 15 nm, 2 to 13 nm, 2 to 11 nm, 2 to 9 nm, 2 to 7 nm, 2 to 5 nm, 2 to 3 nm, 3 to 18 nm, 3 to 16 nm, 3 to 14 nm, 3 to 12 nm, 3 to 11 nm, 3 to 9 nm, 3 to 7 nm, 3 to 5 nm, 3 to 4 nm, 4 to 11 nm, 4 to 9 nm, 4 to 7 nm, 4 to 5 nm, 5 to 10 nm, 5 to 8 nm, 5 to 6 nm, 6 to 10 nm, 6 to 8 nm, 6 to 7.8 nm, 6 to 7.5 nm, 6 to 7.3 nm, 6.3 to 7.3 nm, 6.5 to 7.3 nm, 6.7 to 7.3 nm, or 6.8 to 7.3 nm. According to one embodiment of the present invention, the nitrogen-doped graphene quantum dots may have a diameter of 6.76 to 7.30 nm, but the present invention is not limited thereto.

As used in the present invention, the term "pharmaceutically acceptable salt" includes salts derived from pharmaceutically acceptable inorganic acids, organic acids, or bases.

Examples of suitable acids include hydrochloric acid, hydrobromic acid, sulfuric acid, nitric acid, perchloric acid, fumaric acid, maleic acid, phosphoric acid, glycolic acid, lactic acid, salicylic acid, succinic acid, toluene-p-sulfonic acid, tartaric acid, acetic acid, citric acid, methanesulfonic acid, formic acid, benzoic acid, malonic acid, gluconic acid, naphthalene-2-sulfonic acid, benzenesulfonic acid, and the like. An acid addition salt may be prepared by conventional methods, for example, by dissolving a compound in an excess amount of an aqueous acid solution and precipitating this salt using a water-miscible organic solvent such as methanol, ethanol, acetone, or acetonitrile. Also, the acid addition salt may be prepared by heating equimolar amounts of the compound and an acid or alcohol in water and then evaporating the mixture to dryness, or filtering the precipitated salt by suction.

Salts derived from suitable bases may include alkali metals such as sodium, potassium, and the like, alkaline earth metals such as magnesium and the like, ammonium, and the like but the present invention is not limited thereto. The alkali metal or alkaline earth metal salt may be obtained, for example, by dissolving the compound in an excess amount of an alkali metal hydroxide or alkaline earth metal hydroxide solution, filtering the insoluble compound salt, and then evaporating and drying the filtrate. At this time, it is particularly pharmaceutically suitable to prepare a sodium, potassium, or calcium salt as the metal salt, and the corresponding silver salt may be obtained by reacting an alkali metal or alkaline earth metal salt with an appropriate silver salt (e.g., silver nitrate).

In the present invention, the gene refers to a substance containing nucleotides and a nucleic acid that is a polymer of nucleotides, and may include one or more selected from the group consisting of gDNA, cDNA, pDNA, mRNA, tRNA, rRNA, siRNA, shRNA, miRNA, a DNA-RNA hybrid, and a ribozyme. According to one embodiment of the present invention, the gene may be mRNA and/or pDNA, but the present invention is not limited thereto. The gene may be a gene drug. In this case, the gene drug refers to a negatively charged gene that exhibits medicinal efficacy in treating or preventing one or more diseases.

In the present invention, the nitrogen-doped graphene quantum dots and the gene may be bound at a ratio of 1 to 50:1, 1 to 45:1, 1 to 40:1, 1 to 35:1, 1 to 30:1, 1 to 25:1, 1 to 20:1, 1 to 15:1, 1 to 10:1, 1 to 8:1, 1 to 5:1, 5 to 40:1, 5 to 35:1, 5 to 30:1, 5 to 28:1, 5 to 25:1, 5 to 23:1, 5 to 20:1, 5 to 18:1, 5 to 15:1, 5 to 13:1, 5 to 10:1, 8 to 30:1, 8 to 28:1, 8 to 25:1, 8 to 23:1, 8 to 20:1, 8 to 18:1, 8 to 15:1, 8 to 13:1, 8 to 10:1, 10 to 30:1, 10 to 28:1, 10 to 25:1, 10 to 23:1, 10 to 20:1, 10 to 18:1, 10 to 15:1, or 10 to 13:1. According to one embodiment of the present invention, the nitrogen-doped graphene quantum dots and the gene may be bound at a ratio of 20:1 or 10:1 through electrostatic interaction, but the present invention is not limited thereto.

As used in the present invention, the term "electrostatic interaction" means that opposite charges meet and act through electrostatic attraction.

As used in the present invention, the term "gene delivery" refers to a process of delivering a target gene encoded in DNA or RNA into a cell. In this case, because the gene has a negative charge, the gene may bind to nitrogen-doped graphene quantum dots, which have a surface with a positive charge, through electrostatic attraction (interaction) to form a complex.

As used in the present invention, the term "gene delivery platform" or "gene carrier" refers to any means for delivering genes into cells.

The gene delivery composition according to the present invention may include a pharmaceutically effective amount of the gene alone or may include one or more pharmaceutically acceptable carriers, excipients, or diluents. As described above, the pharmaceutically effective amount refers to an amount sufficient to exhibit the desired physiological or pharmacological activity when the gene is administered to animals or humans. However, the pharmaceutically effective amount may vary appropriately depending on the age, weight, health condition, and gender of a subject to be administered, a route of administration, a treatment period, and the like.

As such, the term "pharmaceutically acceptable" also means that the gene does not usually cause allergic reactions or similar reactions, such as gastrointestinal disorders, dizziness, and the like, when the gene is administered to humans. Examples of such carriers, excipients, and diluents include lactose, dextrose, sucrose, sorbitol, mannitol, xylitol, erythritol, maltitol, starch, gum acacia, alginate, gelatin, calcium phosphate, calcium silicate, cellulose, methyl cellulose, polyvinylpyrrolidone, water, methyl hydroxybenzoate, propyl hydroxybenzoate, talc, magnesium stearate, and mineral oil. Also, fillers, anti-coagulants, lubricants, wetting agents, fragrances, emulsifiers, preservatives, and the like may be further included.

The gene delivery composition according to the present invention may be administered through various routes, including oral, transdermal, subcutaneous, intravenous, or intramuscular administration, and the dosage of the gene may be appropriately selected depending on the route of administration, the age, gender, and weight of a patient, the severity of the patient's condition, and the like. Also, the gene delivery composition of the present invention may be administered in combination with known compounds that can enhance the desired effect of the gene.

Also, the present invention provides a method for preparing nitrogen-doped graphene quantum dots, which includes: subjecting a mixed solution of citric acid and polyethylenimine (PEI) to a hydrothermal reaction. Here, the citric acid and the polyethylamine are used as precursors for nitrogen-doped graphene quantum dots.

The polyethylenimine may be linear polyethylenimine (linear PEI) or branched polyethylenimine (branched PEI), preferably branched polyethylenimine, but the present invention is not limited thereto.

In the present invention, when the linear polyethylenimine is used as a precursor, the linear polyethylenimine may have a molecular weight of 2.5 to 250 kDa, but the present invention is not limited thereto.

In the present invention, when the branched polyethylenimine is used as a precursor, the branched polyethylenimine used may have a molecular weight of 0.6 to 750 kDa, but the present invention is not limited thereto.

As used in the present invention, the term "hydrothermal reaction" refers to a synthesis or denaturation reaction of a substance that occurs in the presence of high-temperature water, especially high-temperature and high-pressure water. According to one embodiment of the present invention, the hydrothermal reaction may be a microwave-assisted hydrothermal reaction, but the present invention is not limited thereto.

The "microwave-assisted hydrothermal reaction" refer to a reaction carried out by microwave irradiation. In this case, the microwaves may be irradiated at an intensity of 300 to 1000 W, 300 to 900 W, 300 to 800 W, 300 to 700 W, 400 to 900 W, 400 to 800 W, 400 to 700 W, 500 to 800 W, 600 to 800 W, 700 to 800 W, or 750 to 800 W. According to one embodiment of the present invention, the microwaves may be irradiated at an intensity of 800 W, but the present invention is not limited thereto.

In the present invention, the citric acid and the polyethylenimine may be mixed in a weight ratio of 1 to 15:1, 1 to 13:1, 1 to 10:1, 1 to 8:1, 1 to 5:1, 1 to 4:1, 1 to 3:1, 1 to 2:1, 2 to 4:1, 2 to 3:1, 3 to 4:1, 3.5 to 4:1, or 4:1, but the present invention is not limited thereto.

In the present invention, the preparation method may further include: removing residual reactants, and the removal of the residual reactants may be performed by dialysis.

In addition, the present invention provides nitrogen-doped graphene quantum dots prepared by the preparation method according to the present invention.

Because polyethylenimine used as a precursor is cytotoxic when delivered alone to cells, polyethylenimine is not suitable for use as a gene delivery platform. However, because the nitrogen-doped graphene quantum dots prepared by the preparation method according to the present invention have a surface with a positive charge, are stable at room temperature, and exhibit low cytotoxicity, the nitrogen-doped graphene quantum dots may be used as the gene delivery platform to deliver genes into cells.

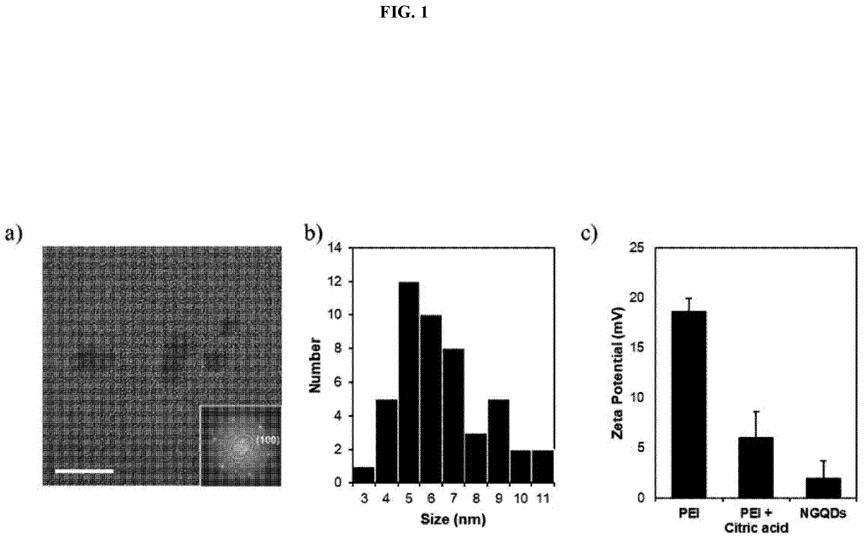

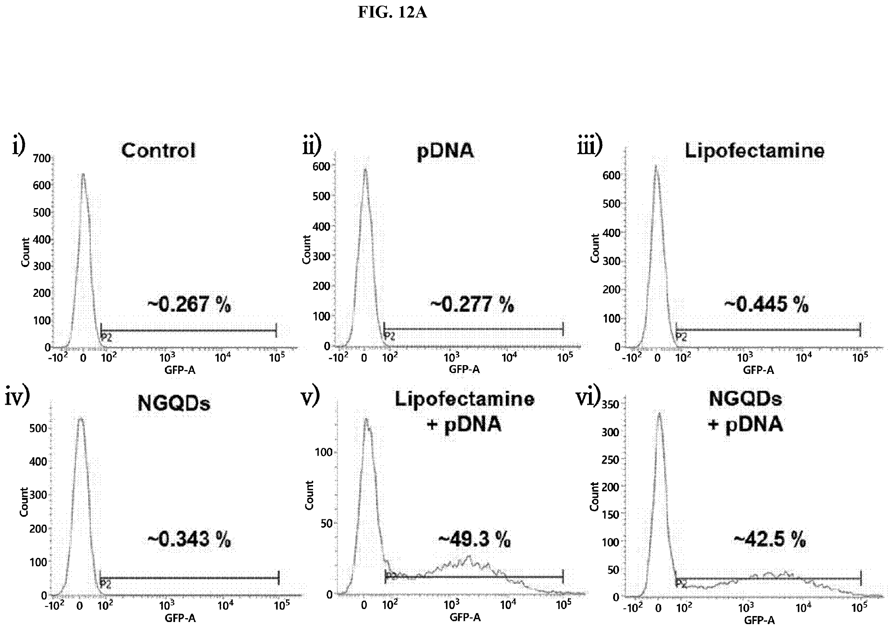

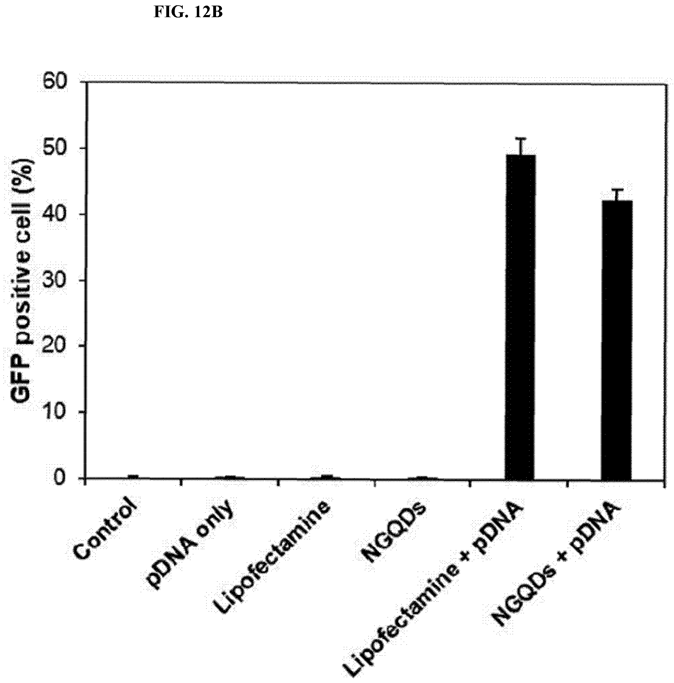

According to one embodiment of the present invention, the nitrogen-doped graphene quantum dots prepared by the preparation method according to the present invention have a low zeta potential value even when polyethylenimine, which is a precursor, has a high zeta potential value. Therefore, the nitrogen-doped graphene quantum dots are expected to be usefully used as a pDNA delivery platform (because pDNA is longer than mRNA, it is strongly bound to make detachment difficult when the zeta potential value is high) (see FIGS. 1 and 12).

In addition, the present invention provides a gene delivery method comprising: administering a composition, which includes the nitrogen-doped graphene quantum dots or a salt thereof bound to a gene to be delivered as an active ingredient, to a subject in need thereof.

Also, the present invention provides a use of the composition comprising the nitrogen-doped graphene quantum dots as the active ingredient for gene delivery.

In addition, the present invention provides a gene delivery method comprising: administering the nitrogen-doped graphene quantum dots or a pharmaceutically acceptable salt thereof to which a target gene is bound to a subject in need thereof.

Additionally, the present invention provides a use of the nitrogen-doped graphene quantum dots or a pharmaceutically acceptable salt thereof for gene delivery.

Further, the present invention provides a use of the nitrogen-doped graphene quantum dots or a pharmaceutically acceptable salt thereof for the preparation of gene carriers.

### [Mode for Invention]

Hereinafter, preferred examples and experimental examples of the present invention are presented in order to aid in understanding the present invention. However, it should be understood that the following examples and experimental examples are provided only to make the present invention easier to understand and are not intended to limit the present invention.

### [EXAMPLES]

### Example 1: Synthesis of nitrogen (N)-doped graphene quantum dots (NGQDs)

200 mg of citric acid (Sigma Aldrich, St. Louis, USA) and 50 mg of polyethylenimine (PEI; Polyscience, Mw 1800, branched, Warrington, USA) were added to 15 mL of distilled water. After sonication for 30 minutes, the clear solution was placed in the center of a microwave (Midea, 800W, MWO-2027, Beijiao, China). A microwave-assisted hydrothermal reaction was repeated approximately 10 times for 30 seconds until the reaction solution turned yellow. The product solution was filtered through 200 nm and 20 nm disc filters (GE Healthcare Life Sciences, Anodise^{™}, Chicago, USA) and dialyzed with a 3.5 kDa dialysis tube (Thermo Fisher Scientific, Snakeskin^{™}, Waltham, USA) for 5 days to remove the residual reactants. The completed solution was freeze-dried for 2 days.

### Example 2: Characterization of NGQDs

The shape and size distribution of NGQDs were analyzed by a Cs-corrected transmission electron microscope (Cs-TEM; JEOL Ltd., JEM-ARM200F, Tokyo, Japan). The zeta potential was measured using a zeta potential analyzer (Malvern Instruments, Zetasizer NanoS, Malvern, UK). The functional groups of NGQDs were characterized using Fourier transform infrared spectroscopy (FT-IR; BRUKER, Vertex-80V, Billerica, USA) and X-ray photoelectron spectroscopy (XPS; Kratos Analytical Ltd., AXIS-His, Manchester, UK). The D and G bands of graphene in the NGQDs were confirmed using a Raman spectrometer (Renishaw, Via Raman microscope, Warton-under-Edge, UK). The absorbance of NGQDs was analyzed using an ultraviolet-visible (UV-Vis) spectrophotometer (Scinco, S-3100, Seoul, Korea). Emission spectra at various excitation wavelengths were obtained using a spectrofluorometer (Jasco Inc., FP-8300, Tokyo, Japan).

### Example 3: Loading capacity

To analyze the ratio of genes to NGQDs, 100 ng of mRNA encoding green fluorescent protein (GFP) (TriLink Biotechnologies, CleanCap EGFP mRNA, San Diego, USA) and pDNA (Addgene, pcDNA3-EGFP, Watertown, USA) were added to various amounts (0, 0.5, 1, 2, and 4 µg) of NGQDs in 20 µL of a 1 × phosphate buffer saline (PBS) solution. The loading process was performed in a 1 mL tube. After incubation for an hour, a series of complexes were mixed with 4 µL of LoadingSTAR^{™} (Dyne Bio Inc., Seongnam, Korea), and the mixture was loaded on a 1% agarose gel. The loading capacity of NGQDs was determined by measuring the intensity of the bands derived from the remaining genes after agarose-gel electrophoresis (ADVANCE, Mupid-2plus, Tokyo, Japan) at 100 V for 30 minutes.

### Example 4: Cell viability analysis

Before transfection, HeLa cells were seeded in a 96-well plate at a density of 5 × 10³ cells for 24 hours and then treated with various concentrations of NGQDs in a complete medium for 24 hours. After the medium was removed, the cells were washed with a 1 × PBS solution and incubated in 90 µL of a serum-free medium with 10 µL of a cell counting kit-8 (CCK-8) solution (Dojindo Molecular Technologies Inc., Rockville, USA). To evaluate the cell viability of the treated cells, the optical density of formazan salt was measured at 450 nm using a microplate absorbance reader (BioTek, Synergy Mx, Winooski, USA), and the background absorbance of the medium was subtracted. This experiment was repeated three times.

### Example 5: Gene transfection efficiency

HeLa cells were seeded in a 24-well plate at a density of 3 × 10⁴ cells. After incubation for 24 hours, the cells were treated with 1 × PBS (Ctrl), mRNA, Lipofectamine^{®} 2000 (Thermo Fisher Scientific), NGQDs, mRNA bound to Lipofectamine^{®} 2000, and mRNA bound to NGQDs in 0.5 mL of a serum-free medium. For the preparation of a complex-containing gene and NGQDs, 3 µL of an mRNA solution (10 µg/mL) was mixed with 6 µL of an NGQD solution (0.1 mg/mL), and 2 µL of a 10 × PBS solution was added along with 9 µL of deionized water. After incubation with the complex for 24 hours, the medium was removed, and the cells were washed with 1 mL of a 1 × PBS solution. Bright-field and fluorescence images (λ_{Ex}/λ_{Em} = 488 nm/507 nm) were obtained by fluorescence microscopy (Nikon Co., Tokyo, Japan) using Meta-morph image analysis software (Molecular Devices, San Jose, USA). For quantitative analysis of transfection, the cells were trypsinized, harvested, and then suspended in 0.5 mL of a 1 × PBS solution. The fluorescence intensity of the sample was analyzed by flow cytometry (BD Biosciences, BD FACSLyric, USA).

### [EXPERIMENTAL EXAMPLES]

### Experimental Example 1: Characterization of NGQDs

To deliver genes such as mRNA and pDNA into cells, GQDs having a positive charge that can interact with genes through electrostatic interactions were synthesized. GQDs were synthesized by a microwave-assisted hydrothermal reaction using PEI and citric acid as precursors, and the synthesized GQDs were named nitrogen-doped graphene quantum dots (NGQDs).

### 1-1. Shape, size, and surface charge of NGQDs

TEM observation was performed to analyze the shape and size of the prepared NGQDs. As a result, as shown in FIGS. 1A and 1B, it was confirmed that the NGQDs were nanoparticles having an overall diameter distribution of 3 to 11 nm and an average diameter of 7.03 nm ± 0.27 nm. Also, as shown in FIG. 1C, zeta potential measurements provided additional surface charge information of NGQDs having a surface environment charged with a positive charge of 1.91 ± 1.77 mV in deionized water. Through this positive charge, the NGQDs were able to electrostatically interact with genes with a negatively charged phosphate backbone.

### 1-2. Optical properties of NGQDs

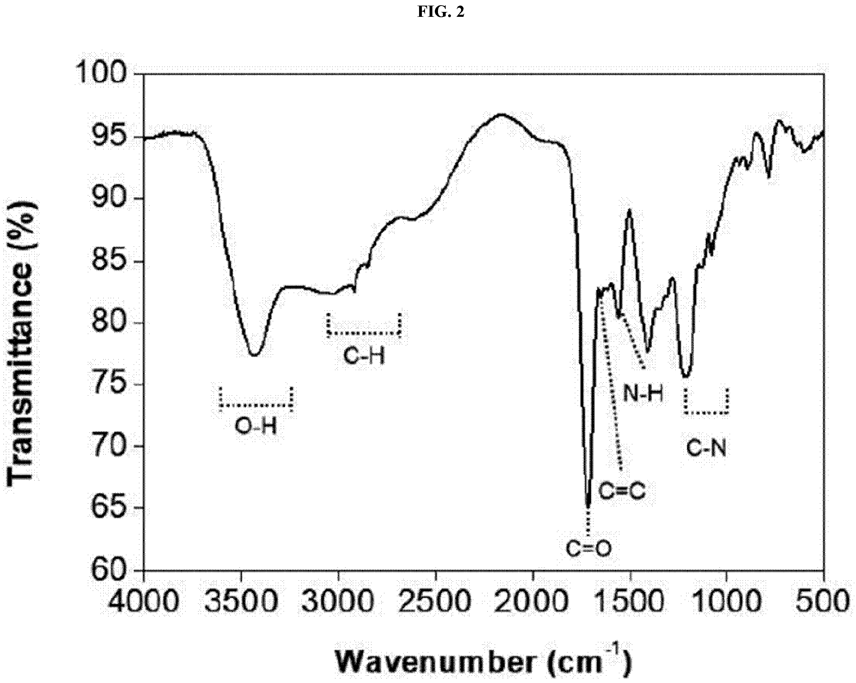

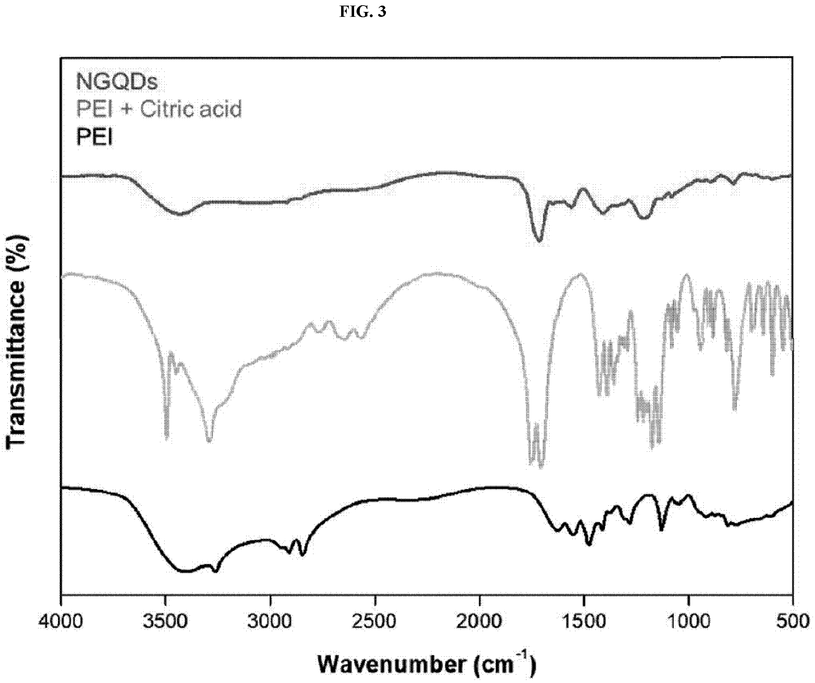

FT-IR and XPS were performed to analyze the functional groups of NGQDs. As a result of FT-IR analysis, as shown in FIG. 2, representative peaks of NGQDs were observed at 1,720 and 1,650 cm⁻¹, which were interpreted as C=O stretching and C=C stretching vibrations in carboxylic acid and aromatic groups, respectively. Also, peaks were clearly observed at 1,550 and 1,000 to 1,250 cm⁻¹, which correlated with the C-N bonding of the amine groups as well as the N-H bending of the primary and secondary amine groups. The FT-IR spectra of the precursors are shown in FIG. 3.

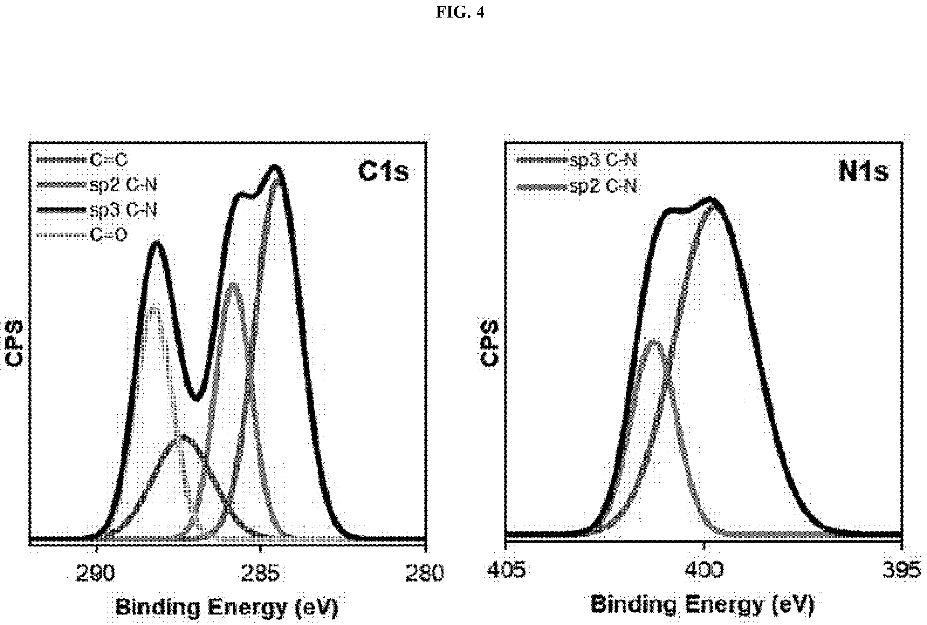

As a result of XPS analysis, as shown in FIG. 4, it can be seen in the C1s and N1s spectra that the NGQDs included aromatic sp2 C=C, sp2 C-N, sp3 C-N, and carboxyl groups, which corresponded to the peaks of 284.5, 285.7, 287.5, and 288.6 eV.

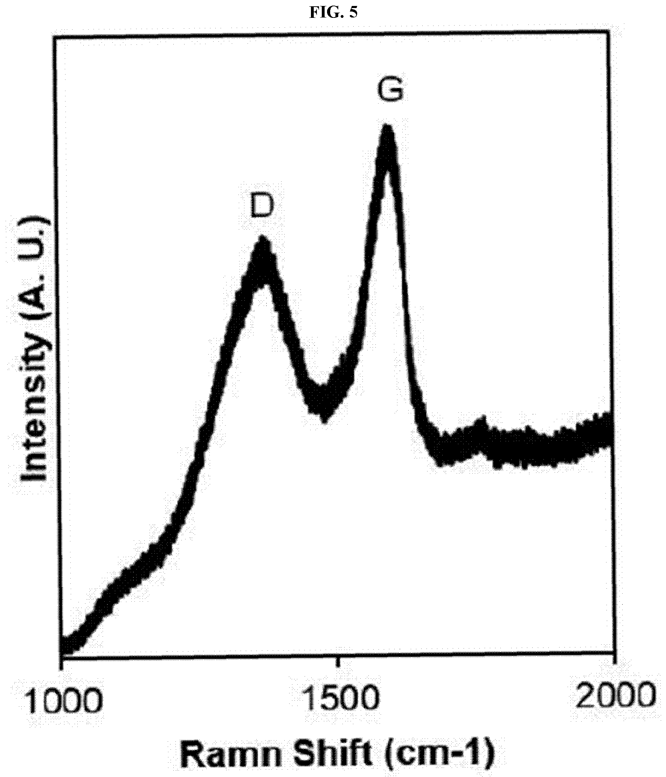

In addition, as shown in FIG. 5, D and G bands were observed at 1,350 cm⁻¹ and 1,580 cm⁻¹, respectively, as characteristic peaks of graphene in the Raman spectrum. From the above results, it can be seen that the NGQDs were composed of aromatic sp2 domains and various functional groups such as carboxylic acid, an amine group, and the like.

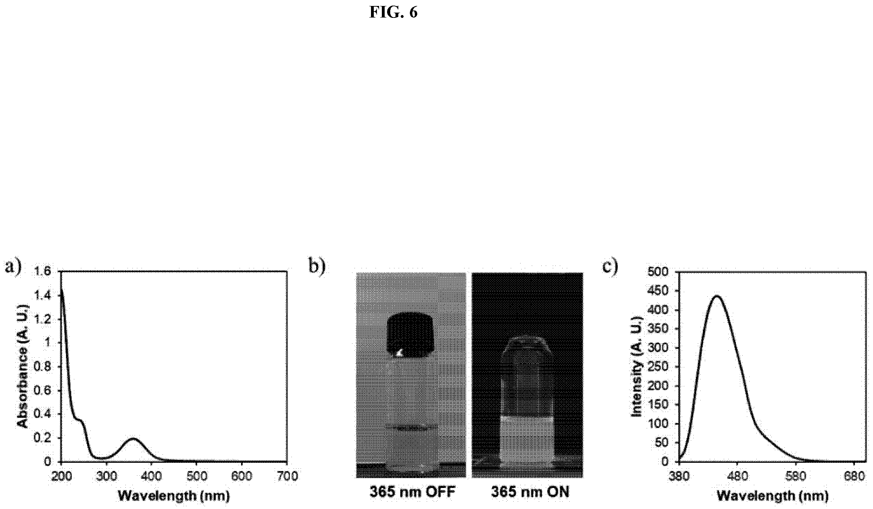

As shown in FIG. 6A, the NGQDs exhibited UV-Vis absorption peaks at 250 and 350 nm, which corresponded to the ππ* transition of aromatic sp2 C=C and the n-π* transition of carbonyl C=O, respectively, indicating that the NGQDs were composed of an aromatic sp2 C=C domain and a carbonyl group.

Also, as shown in FIGS. 6B and 6C, the NGQDs emitted blue fluorescence (Em, max = 432 nm) at 365 nm excitation.

From the above overall characterization data of NGQDs, it can be seen that the NGQDs had characteristic properties of GQDs (graphite core containing various functional groups and a positive charge required to interact with genes).

### Experimental Example 2: Loading capacity of NGQDs

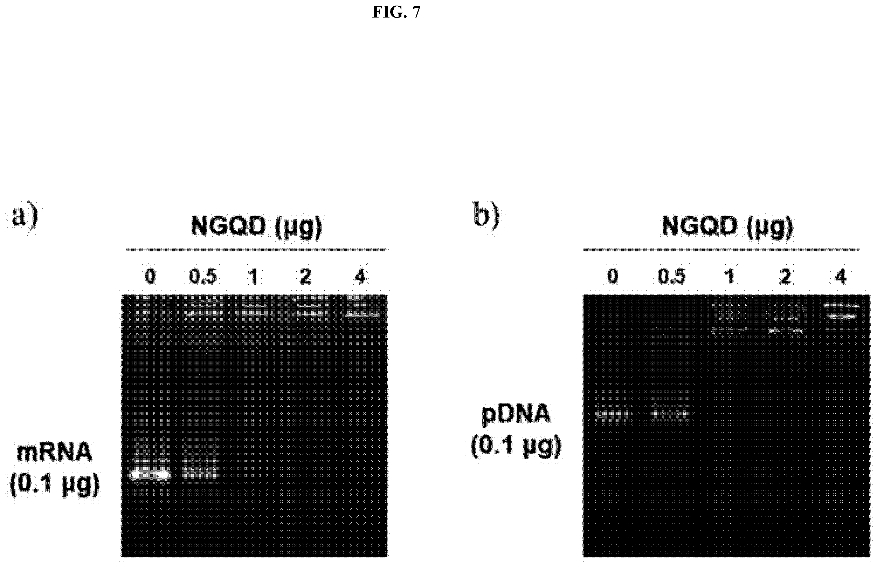

To evaluate the gene loading capacity of NGQDs, the NGQDs were mixed with two types of genes in a 1 × PBS solution and then incubated at room temperature. As shown in FIGS. 7A and 7B, the agarose-gel electrophoresis results showed that the columns of 1 µg and 0.5 µg NGQDs for 0.1 µg of mRNA and 0.1 µg of pDNA in agarose gel showed incomplete band shifts. Therefore, the equivalent amounts of NGQDs for complete loading could be assumed to be 1 to 2 µg for 0.1 µg of mRNA and 0.5 to 1 µg for 0.1 µg of pDNA, respectively.

While mRNA, which is single-stranded and has a linear structure, has a basic backbone structure in which the sugar moiety is ribose, and has an OH group at carbon 2, plasmid DNA (pDNA) is a double-stranded, cyclic DNA and has an H group at carbon 2. Due to these structural differences, pDNA has fewer molecules than mRNA even when the pDNA has the same mass as mRNA, and because there is an H group instead of an OH group at carbon 2, pDNA is expected to have a relatively low charge amount, which results in a difference in the equivalent amount of NGQDs for mRNA and pDNA.

Because NGQDs charged with a positive charge can interact with genes through an electrostatic force, the NGQDs may form complexes with the genes by simple mixing at room temperature.

### Experimental Example 3: Cytotoxicity of NGQDs

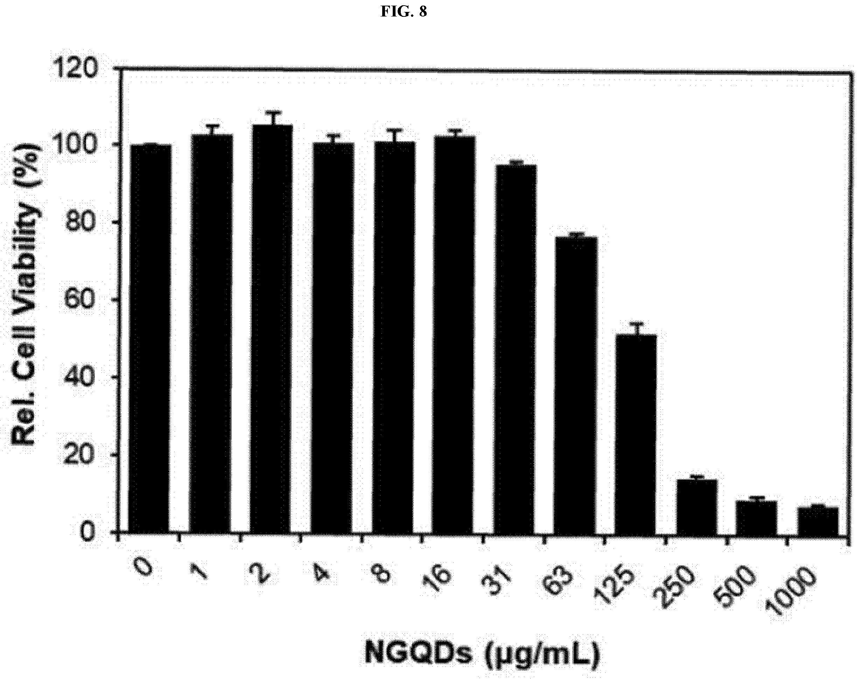

Before *in vitro* gene transfection with NGQDs, the cytotoxicity of NGQDs was confirmed using a CCK-8 assay kit. Specifically, HeLa cells were treated with various concentrations of NGQDs from 1 µg/mL to 1000 µg/mL in a complete medium for a day. As shown in FIG. 8, it can be seen that the NGQDs showed toxicity in a dose-dependent manner, and a decrease in cell viability was observed at a concentration of 63 µg/mL.

### Experimental Example 4: Transfection efficiency of NGQDs

### 4-1. mRNA

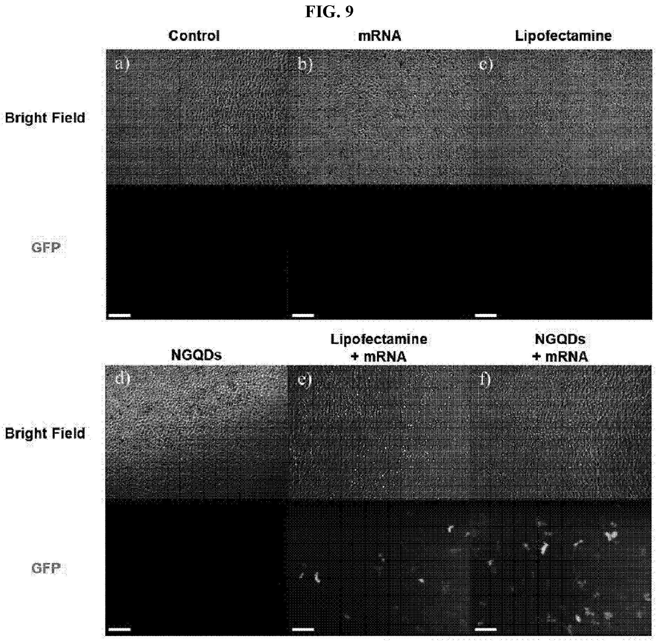

To evaluate the transfection efficiency of NGQDs, mRNA was delivered into HeLa cells containing the NGQDs. As shown in FIG. 7A, because the NGQDs were able to form a complex with mRNA at a ratio (wt/wt) of 20:1, 30 ng of mRNA encoding GFP formed a complex with 600 ng of NGQDs before transfection. Lipofectamine 2000, which is a representative transfection agent that is used to form various genes and liposomes, was used as a positive control. After incubation at room temperature for an hour, the experimental groups were divided into an NGQDs-mRNA complex-treated group, 1 × PBS (control), an mRNA-alone-treated group, a Lipofectamine-alone-treated group, an NGQDs-alone-treated group, and a Lipofectamine-mRNA complex-treated group, and HeLa cells were treated for 24 hours. To visually confirm the transfection of each treatment group, green fluorescence was observed using fluorescence microscopy. As a result, as shown in FIG. 9, it can be seen that the number of cells expressing GFP in the NGQDs-mRNA complex-treated group was similar to that of the group treated with Lipofectamine as a transfection agent, indicating that the NGQDs forming a complex with the gene successfully entered the cells. Also, the NGQDs formed complexes with mRNA and pDNA at 10- to 20-fold the amount of mRNA or pDNA, indicating that multiple NGQDs wrapped around an mRNA or DNA molecule and deliver the molecule to the surrounding cell membrane, followed by cellular uptake. Furthermore, although some preferential interactions of positively charged NGQDs with cell membranes have been reported previously, it could be assumed that the neutralized charge complex between NGQDs and the gene enables cellular uptake. Meanwhile, even though the NGQDs had a fluorescence emission of approximately 500 nm under excitation at 360 nm, which is a wavelength similar to the GFP emission, the excitation range of the NGQDs was 280 to 430 nm, not the GFP excitation wavelength (approximately 488 nm). As a result, as shown in FIG. 9D, the fluorescence of the NGQDs could not be visualized in a fluorescence microscopy image. Therefore, the fluorescence shown in FIG. 9F did not come from the fluorescence of NGQDs. From the above results, it can be seen that the NGQDs successfully delivered mRNA to cells and released the mRNA for translation.

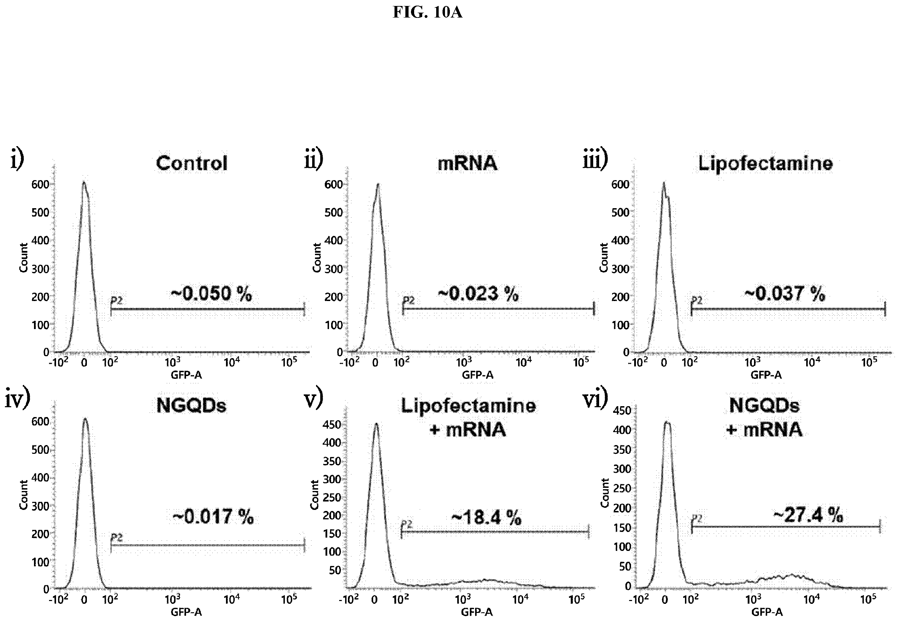

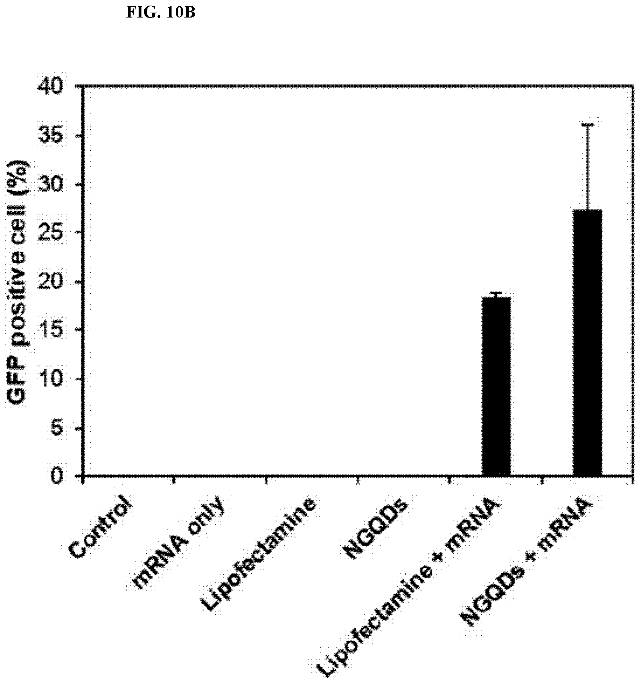

Flow cytometry was performed to quantify the transfection efficiency of each treatment group. As a result, as shown in FIG. 10, similar to the results of fluorescence microscopy, GFP-expressing cells were not detected in the control, the mRNA-alone-treated group, the Lipofectamine-alone-treated group, and the NGQDs-alone-treated group. In particular, the fluorescence of NGQDs at approximately 500 nm was not affected in flow cytometry. Although the NGQDs-mRNA complex-treated group and the Lipofectamine-mRNA complex-treated group had similar fluorescence images, the NGQDs-mRNA complex-treated group showed up to 50% improved transfection compared to the Lipofectamine-mRNA complex-treated group, which was a positive control in quantitative analysis. From the above results, it can be seen that the NGQDs have great potential as an mRNA delivery platform for vaccination or gene therapy.

### 4-2. pDNA

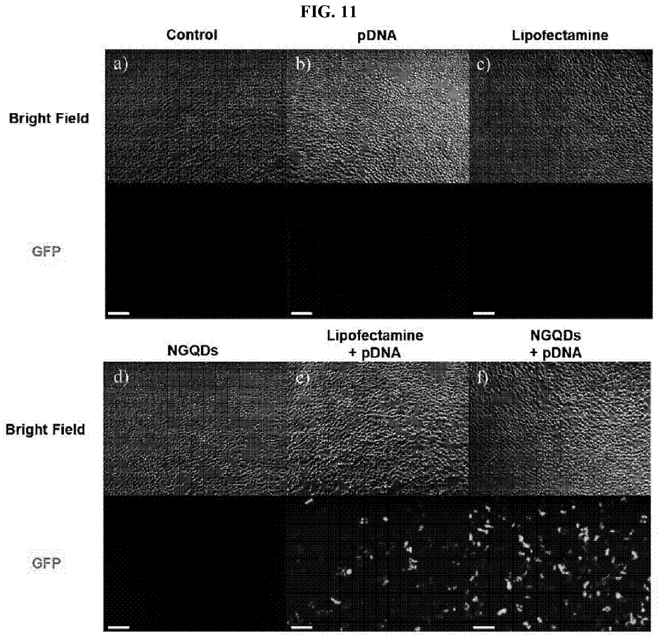

In addition to mRNA, pDNA transfection was performed using NGQDs. In the same way as mRNA, pDNA encoding GFP and forming a complex with NGQDs was prepared by mixing at room temperature. After incubation for an hour, HeLa cells were treated for 24 hours. After treatment, fluorescence microscopy images showed that the NGQDs had the best performance as a pDNA delivery platform. As shown in FIG. 11, the NGQDs-pDNA complex-treated group showed strong green fluorescence similar to the Lipofectamine-pDNA complex-treated group in the fluorescence microscopy images.

Flow cytometry was performed to quantify the transfection efficiency of each treatment group. As a result, as shown in FIG. 12, it was confirmed that the transfection efficiency (approximately 42.5%) of the NGQDs-pDNA-treated group was similar to the transfection efficiency (approximately 49.3%) of the Lipofectamine-pDNA-treated group. It was assumed that the discrepancy between the flow cytometry results in FIG. 12 and the fluorescence microscopy image in FIG. 11 was due to the difference in fluorescence intensity of the transfected cells. Because cells treated with NGQDs-pDNA tended to show strong fluorescence in flow cytometry results, the fluorescence images appeared brighter, as shown in FIGS. 11E and 11F.

As confirmed above, although there were differences in the intracellular locations where mRNA and pDNA act, the NGQDs delivered both mRNA and pDNA to the cells, and the delivered mRNA and pDNA functioned successfully. From the above results, it can be seen that the NGQDs were a suitable delivery platform capable of transfecting various types of genes regardless of the intracellular compartment in which the genes act.

The above description of the present invention is for illustrative purposes. Therefore, those skilled in the art to which the present invention pertains will appreciate that the present invention can be embodied in other specific forms without changing the technical spirit or essential features of the present invention. Therefore, it should be understood that the embodiments described above are illustrative in all respects and not restrictive.

### [Industrial Applicability]

Nitrogen-doped graphene quantum dots of the present invention exhibit excellent safety and low cytotoxicity at room temperature, and have a surface with a positive charge, and thus can bind to negatively charged genes through electrostatic attraction to deliver the genes into cells. Also, the nitrogen-doped graphene quantum dots of the present invention can be usefully used for a gene delivery platform for gene therapy because the nitrogen-doped graphene quantum dots of the present invention exhibits excellent gene delivery efficiency and transfection efficiency. Therefore, the present invention is industrially applicable.

## Claims

1. A gene delivery composition comprising nitrogen (N)-doped graphene quantum dots (NGQDs) or a pharmaceutically acceptable salt thereof as an active ingredient.

2. The gene delivery composition or a pharmaceutically acceptable salt thereof of claim 1, wherein the nitrogen-doped graphene quantum dots have a zeta potential ranging from 0.1 to 20 mV.

3. The gene delivery composition of claim 1, wherein the nitrogen-doped graphene quantum dots have a diameter of 1 to 20 nm.

4. The gene delivery composition of claim 1, wherein the nitrogen-doped graphene quantum dots and the gene are bound at a ratio of 1 to 50:1.

5. The gene delivery composition of claim 1, wherein the nitrogen-doped graphene quantum dots and the gene are bound through electrostatic interaction.

6. The gene delivery composition of claim 1, wherein the gene is one or more selected from the group consisting of gDNA, cDNA, pDNA, mRNA, tRNA, rRNA, siRNA, shRNA, miRNA, a DNA-RNA hybrid, and a ribozyme.

7. A method of preparing nitrogen-doped graphene quantum dots, comprising:
subjecting a mixed solution of citric acid and polyethylenimine (PEI) to a hydrothermal reaction.

8. The method of claim 7, wherein the hydrothermal reaction is carried out by microwave irradiation.

9. The method of claim 7, wherein the citric acid and the polyethylenimine are mixed in a weight ratio of 1 to 10:1.

10. Nitrogen-doped graphene quantum dots prepared by the preparation method of any one of claims 7 to 9.

11. The nitrogen-doped graphene quantum dots of claim 10, wherein the nitrogen-doped graphene quantum dots have a zeta potential ranging from 0.1 to 20 mV.

12. The nitrogen-doped graphene quantum dots of claim 10, wherein the nitrogen-doped graphene quantum dots have a diameter of 1 to 20 nm.

13. A gene delivery method comprising:
administering nitrogen-doped graphene quantum dots or a pharmaceutically acceptable salt thereof to which a target gene is bound to a subject in need thereof.

14. A use of the nitrogen-doped graphene quantum dots or a pharmaceutically acceptable salt thereof for gene delivery.

15. A use of the nitrogen-doped graphene quantum dots or a pharmaceutically acceptable salt thereof for the preparation of gene carriers.
